# EUROPEAN PATENT APPLICATION

(11) **EP 0 638 330 A1**
(43) Date of publication of application: **15.02.1995**
(21) Application number: 94112261.6
(22) Date of filing: 05.08.1994
(51) Int. Cl.: A61N 1/32

(54) **Electrotherapy transducer**

(30) Priority: 06.08.1993 IT TO930596
(71) Applicant: OT. ENGINEERING S.r.l., I-10086 Rivarolo Canavese (IT)
(72) Inventor: Ottino, Domenico, I-10086 Rivarolo Canavese (IT)
(74) Representative: Prato, Roberto

(57) **Abstract**

An electrotherapy transducer (1) wherein an insulating sheet (6) of flexible plastic material supports a number of square electrodes (3a, 3b) arranged in the form of matrix and presenting equally spaced (D) adjacent straight edges; the transducer (1) being connected to a signal source for supplying adjacent electrodes (3a, 3b) with electric signals of opposite polarity.

## Description

The present invention relates to an electrotherapy transducer.

Electrotherapy devices are known wherein a signal source supplies a transducer connected to a surface portion of the human body with a periodic electric signal of a given amplitude and waveform, for producing a therapeutic current in the body portion close to the transducer.

Known transducers generally comprise two (or more) normally flat metal electrodes which are placed manually on to the surface of the patient's body and strapped or tied in place. Being adjusted only roughly by hand, the distance between the adjacent edges of the electrodes is seldom constant, so that the current flowing between the electrodes often differs in intensity, thus resulting in nonuniform current distribution and impaired therapeutic efficiency of the transducer as a whole.

It is an object of the present invention to provide an electrotherapy transducer designed to overcome the drawbacks typically associated with known transducers.

According to the present invention, there is provided a transducer as described in Claim 1.

The present invention will be described with reference to the accompanying drawings, in which:
Figure 1 shows a schematic plan view of a first embodiment of a transducer in accordance with the present invention;
Figure 2 shows a plan view, with parts removed for clarity, of a further embodiment of a transducer in accordance with the present invention;
Figure 3 shows a section along line III-III in Figure 2.

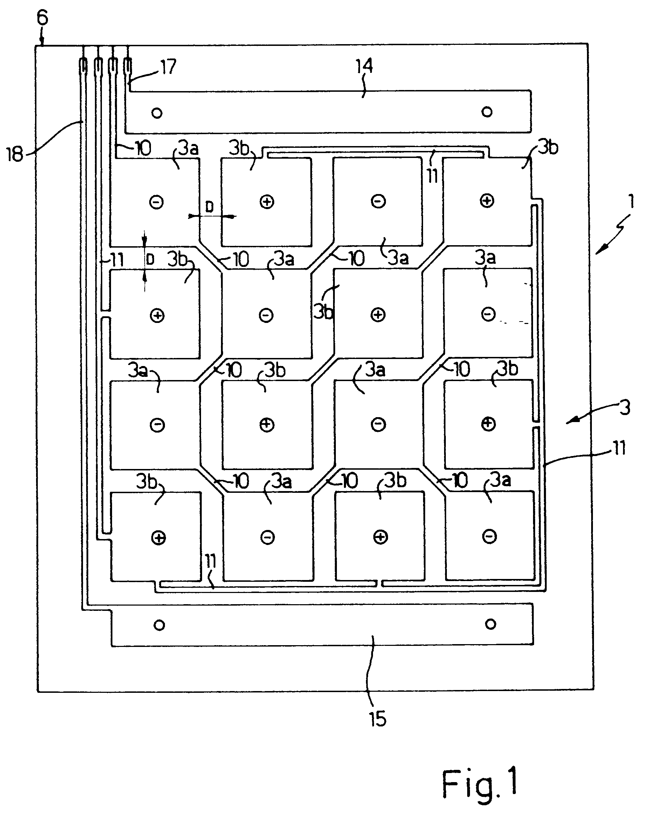

Number 1 in Figure 1 indicates an electrotherapy transducer comprising sixteen square electrodes 3 arranged on a flexible support 6 conveniently formed from a rectangular sheet of insulating material (e.g. rubber or soft plastic) and which is applied to a portion of the patient's body (not shown).

More specifically, electrodes 3 are arranged on sheet 6 in such a manner as to form an orderly square matrix structure, and present straight adjacent edges separated by a constant distance D.

Electrodes 3 are made of metal (e.g. stainless steel, pure aluminium, titanium, etc.) or good electrically conductive synthetic material.

Transducer 1 also comprises a first electric connecting line 10 connecting eight electrodes 3a; and a second connecting line 11 connecting eight electrodes 3b adjacent to and equally spaced in relation to electrodes 3a. Each row and each column in the matrix structure is thus composed of alternate electrodes 3a and 3b connected respectively to lines 10 and 11.

Transducer 1 also comprises two auxiliary elongated rectangular electrodes 14, 15 located on either side of, and with their longer sides parallel to the edges of, the matrix structure, and connected to respective electric lines 17, 18 supported on sheet 6.

In actual use, transducer 1 is applied to a portion of the patient's body, e.g. a thigh, to which it is secured by means of devices not shown (e.g. straps or ties) for securing the supporting sheet to the body portion with electrodes 3a, 3b, 14, 15 contacting the patient's skin.

Lines 10, 11 are then input-connected to a known electronic electrotherapy device (not shown) presenting a first signal source (not shown) for supplying lines 10, 11 with periodic electric signals of opposite polarity.

Adjacent equally spaced electrodes 3a, 3b are thus supplied with signals of opposite polarity, and therapeutical surface currents are formed in the surface portions of the patient's body interposed between electrodes 3a, 3b.

The intensity of the surface currents is noticeably constant and the spatial distribution of the currents homogeneous on account of said orderly arrangement of electrodes 3a, 3b.

As such, for a given signal source, the therapeutic effect of transducer 1 is greatly enhanced as compared with that of known transducers.

In particular, tests have shown that transducer 1 according to the present invention may be used effectively for the treatment of cellulitis.

For further improving distribution of the surface electric current, medium or low conductivity material (e.g. square sheets of porous material soaked in electrolyte) may be inserted between electrodes 3a, 3b and the surface for treatment.

Auxiliary electrodes 14, 15 are connected to a second signal source (not shown) by which they are supplied with periodic signals of opposite polarity and greater amplitude as compared with the signal supplied to lines 10, 11.

A current flow is thus formed between electrodes 14, 15, which flows deeply inside the treated portion of the patient's body and has a therapeutic effect on the muscles.

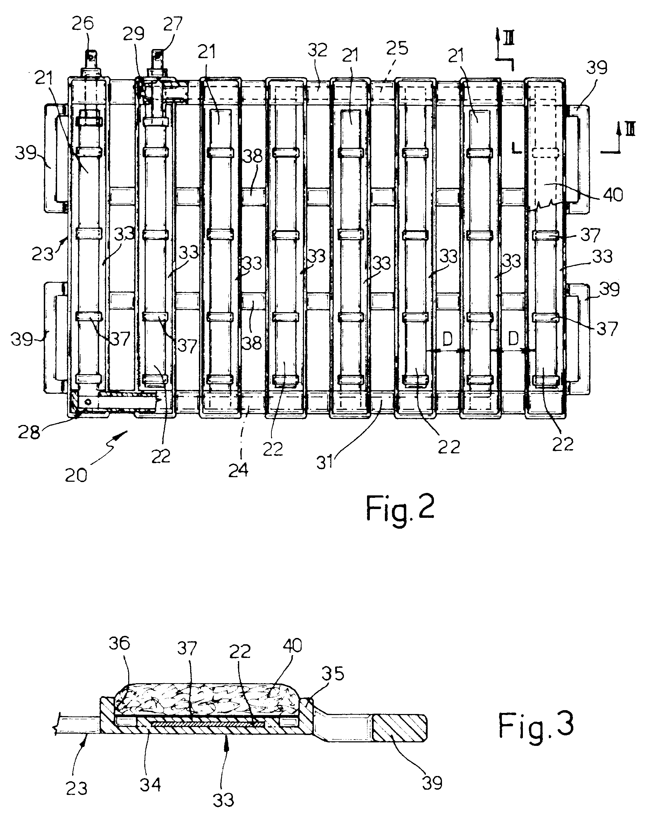

Number 20 in Figure 2 indicates a further embodiment of the transducer according to the present invention.

Transducer 20 comprises a first number of electrodes 21 and a second number of electrodes 22 arranged alternately on a flexible support 23.

More specifically, electrodes 21, 22 consist of elongated rectangular blades of conductive metal, and are arranged side by side, parallel to one another, and equally spaced by a constant distance D. Electrodes 21 are connected, e.g. welded, at one end to a first supply line 24 perpendicular to the electrodes and also consisting of an elongated metal blade extending along one edge of support 23.

Similarly, electrodes 22 are connected at the opposite end to a second supply line 25 parallel to line 24 and extending along the opposite edge of support 23.

One electrode 21 and one electrode 22 are connected to respective terminals 26, 27 in turn connected to an electronic electrotherapy device with a signal source (not shown) for supplying the two numbers of electrodes with periodic electric signals of opposite polarity.

Together with the respective supply lines, said numbers of electrodes 21, 22 thus constitute respective substantially comb-shaped conductors 28, 29 coplanar with and rotated 180 degrees in relation to each other, so that respective electrodes 21, 22 are arranged alternately and equally spaced in relation to one another.

Support 23 is conveniently molded from plastic insulating material, and is conveniently co-molded on to preassembled conductors 28, 29 so as to partially incorporate them.

More specifically, support 23 comprises, integrally, a pair of parallel longitudinal lateral strips 31, 32 incorporating respective supply lines 24, 25; and a number of transverse elements 33 supporting respective electrodes 21, 22 and connected integrally at the ends to strips 31, 32.

As shown clearly in Figure 3, each transverse element 33 presents a flat bottom wall 34 on which respective electrode 21 or 22 rests; and a raised peripheral edge 35 extending about the entire periphery of element 33 so as to define an elongated rectangular seat 36. Each element 33 also comprises a number of thin transverse strips 37 formed integrally during molding and which provide for clamping electrode 21 or 22 on to bottom wall 34.

Support 23 also comprises a pair of intermediate longitudinal strips 38 for integrally connecting supporting elements 33.

Finally, support 23 conveniently presents loops 39 for straps (not shown) for securing transducer 20 to the portion of the patient's body undergoing treatment.

Seats 36 of supporting elements 33 house respective strips 40 (one of which is shown partially in Figure 2) of soft, porous, e.g. fibrous nonwoven, material, for covering electrodes 21, 22 and which may be impregnated with conductive fluid or gel.

Clearly, changes may be made to transducers 1, 20 as described and illustrated herein without, however, departing from the scope of the present invention.

For example, the matrix structure of transducer 1 may be rectangular and comprise a number of electrodes other than that shown.

Also, changes may be made to the shape of the electrodes which may, for example, be circular.

## Claims

1. An electrotherapy transducer (1), characterized in that it comprises:
a number of electrodes (3a, 3b; 21, 22) of conductive material; and
flexible sheet supporting means (6; 23) for supporting said electrodes (3a, 3b; 21, 22) and applied to a portion of the patient's body undergoing electrotherapy;
said electrodes (3a, 3b; 21, 22) being arranged on said supporting means (6; 23) so as to form a substantially orderly structure wherein the adjacent electrodes (3a, 3b; 21, 22) are separated by a notably constant distance (D).

2. A transducer as claimed in Claim 1, characterized in that it comprises first means (10; 24) for electrically connecting a first number of said electrodes (3a; 21); and second means (11; 25) for electrically connecting a second number of said electrodes (3b; 22);
adjacent electrodes (3a, 3b; 21, 22) being connected respectively to said first (10; 24) and second (11; 25) means.

3. A transducer as claimed in Claim 1 or 2, characterized in that said electrodes (3a, 3b) are arranged on said supporting means (6) so as to form a matrix structure.

4. A transducer as claimed in Claim 3, characterized in that it comprises two elongated auxiliary electrodes (14, 15) on either side of said matrix structure.

5. A transducer as claimed in Claim 4, characterized in that said auxiliary electrodes (14, 15) are in the form of an elongated rectangle.

6. A transducer as claimed in any one of the foregoing Claims, characterized in that said electrodes (3a, 3b) are square shaped.

7. A transducer as claimed in Claim 1 or 2, characterized in that said electrodes (21, 22) are elongated in shape and arranged parallel and side by side; the electrodes (21) in said first number alternating with the electrodes (22) in said second number.

8. A transducer as claimed in Claim 7, characterized in that said first and second electric connecting means (24, 25) connect respective ends of said first number of electrodes (21) and respective opposite ends of said second number of electrodes (22).

9. A transducer as claimed in Claim 7 or 8, characterized in that said supporting means comprise a support (23) of plastic material co-molded on to said electrodes (21, 22) and said electric connecting means (24, 25).
